Europäisches Patentamt

(19) European Patent Office

Office européen des brevets

(11) Publication number: **0 252 588**
**A2**

(12) **EUROPEAN PATENT APPLICATION**

(21) Application number: **87304133.9**

(22) Date of filing: **08.05.87**

(51) Int. Cl.³: **C 12 P 21/02**
**C 07 K 1/14, A 61 K 39/015**

(30) Priority: **12.05.86 US 861810**

(43) Date of publication of application:
**13.01.88 Bulletin 88/2**

(84) Designated Contracting States:
**AT BE CH DE ES FR GB GR IT LI LU NL SE**

(71) Applicant: SMITHKLINE BECKMAN CORPORATION
One Franklin Plaza P O Box 7929
Philadelphia Pennsylvania 19103(US)

(72) Inventor: Dephillips, Peter Arletti
577 Hidden Valley Road
King of Prussia Pennsylvania 19406(US)

(72) Inventor: Wasserman, Gail Marie Folena
9 Brentwood Place
Holland Pennsylvania 18966(US)

(72) Inventor: Sitrin, Robert David
237 Emerson Drive
Lafayette Hill Pennsylvania 19444(US)

(72) Inventor: Zabriskie, Dane William
401 Sunny Brook Lane
West Chester Pennsylvania 19382(US)

(74) Representative: Giddings, Peter John, Dr. et al,
Smith Kline & French Laboratories Ltd. Corporate Patents
Mundells
Welwyn Garden City Hertfordshire AL7 1EY(GB)

(54) Process for the isolation and purification of P. falciparum CS protein expressed in recombinant E. coli, and its use as a vaccine.

(57) Immunogenic polypeptides derived from the repeat region of the circumsporozoite protein of *Plasmodium falciparum* are purified by a series of precipitation and chromatographic procedures.

EP 0 252 588 A2

Croydon Printing Company Ltd

TITLE MODIFIED
S??

- 1 -

# PROCESS FOR ISOLATING AND PURIFYING P. FALCIPARUM
## C S PROTEIN VACCINE EXPRESSED IN RECOMBINANT E. COLI

This invention relates to a process for producing purified polypeptide, expressed in recombinant E. coli, having therapeutic utility as a vaccine for protecting humans against infection by Plasmodium falciparum, the infective agent of malaria.

In European Patent Application, EP-A-192,626 by Ballou et al. (U.S. Patent Application Serial No. 699,116), which is incorporated by reference, there is disclosed and claimed an immunogenic polypeptide capable of conferring immunity in mammals to infection by P. falciparum, and to a vaccine comprising the immunogenic polypeptide. The immunogenic polypeptide comprises four or more tandem repeat units of the P. falciparum CS protein. The P. falciparum repeat unit is a tetrapeptide having the following sequence:
Asparagine (Asn) - alanine (Ala) - Asn-proline (Pro) - .
In European Patent Application, EP-A-191,748 by Gross et al., (U.S. Patent Application Serial No. 699,115), which is incorporated by reference, there is described and claimed an E. coli expression vector having a coding sequence for all or a portion of the repeat unit of the CS protein P. falciparum, as well as E. coli transformed with the expression vector and a process for purifying the immunogenic polypeptide from a producing E. coli culture.

A persistent problem in the manufacture of new drugs and biologicals produced by recombinant-DNA technology is the recovery of the product in sufficiently pure form for its intended use. Vaccines, for example, must be sufficiently free of various contaminants of cellular origin, including polypeptides, proteins, nucleic acids and pyrogenic materials to prevent the development of undesirable immune or toxic reactions to such contaminants. Isolation and purification techniques must be designed to specifically eliminate microbial nucleic acid contamination, undesirable antigenic substances and pyrogenic materials, i.e. materials which elicit febrile response in the recipient. Pyrogenic materials are typically bacterial endotoxins, such as lipopolysaccharides.

According to the purification procedure disclosed in EP-A-191,748 and EP-A-192,626, referred to above, polypeptides derived from the P. falciparum CS protein can be separated from other polypeptides by heating clarified cell extract to about 80°C following addition of a detergent to maintain solubility of the protein. Heating to 80°C for at least about 4 minutes causes nearly all undesired bacterial polypeptides and proteins to precipitate without substantially decomposing the desired immunogenic polypeptide. The precipitated bacterial polypeptides and proteins can thus be pelleted by centrifugation and removed. Young et al., Science 228:958 (1985), report on certain of the peptides disclosed in EP-A-192,626 and EP-A-191,748 and purification thereof by ammonium sulfate precipitation and reversed-phase chromatography.

Further efforts to develop a production scale isolation and purification procedure for polypeptides derived from P. falciparum CS protein have shown that the above-described heat treatment, while effective for separating most cellular polypeptide contaminants, does not satisfactorily remove other contaminants such as pyrogens and nucleic acids. This problem is particularly acute in polypeptides derived from P. falciparum CS protein having relatively long basic "tails", such as the $Rtet_{32}$ polypeptides, which comprise at least four repeats with a 32 amino acid "tail" rich in arginine. Because of the basic character of the "tail", the DNA is tightly held in the complex. It has also been found that the concentration of endotoxins in the heat-treated cell extract is also undesirably high.

## SUMMARY OF THE INVENTION

In accordance with the present invention, there is provided a process for isolating and purifying a polypeptide, comprising four or more tandem repeat units of the P. falciparum CS protein, from a partially purified cell lysate derived from a recombinant E. coli host cell culture, wherein the lysate contains protein, nucleic acid and pyrogenic contaminants of cellular origin. The process comprises a series of selective precipitation steps followed by two chromatographic steps, ion exchange chromatography and reversed-phase chromatography.

More particularly, the invention is a process for purifying an immunogenic polypeptide, comprising four or more tandem repeat units of the P. falciparum CS protein, from a clarified cell lysate from a recombinant E. coli host cell culture which comprises:

(a) selectively precipitating bacterial contaminants;

(b) selectively precipitating the immunogenic polypeptide from the supernatant of step (a);

(c) resolubilizing the precipitate from step (b) containing the immunogenic polypeptide and selectively precipitating bacterial contaminants from the solution;

(d) contacting the solution of the immunogenic polypeptide with an ion exchange support and collecting fractions which contain the polypeptide; and

(e) contacting the solution of the immunogenic polypeptide with a solid, hydrophobic support whereby the polypeptide is adsorbed to the support, eluting the polypeptide from the support with a polar organic solvent and collecting fractions which contain the purified poplypeptide.

In a preferred embodiment, the invention is a process for isolating and purifying $R32NS1_{81}$ which comprises.

a) disrupting the cells and separating the cellular debris from said suspension to provide a clarified cell extract containing the peptide $R32NS1_{81}$ together with undesired polypeptides, proteins, DNA and endotoxins;

b) treating the clarified extract with polyethyleneimine so as to precipitate undesired bacterial contaminants and thereafter separating the precipitated bacterial contaminants from the supernatant containing the peptide $R32NS1_{81}$;

c) adding ammonium sulfate to the supernatant containing $R32NS1_{81}$ to a concentration of about 20% to about 40% of saturation, precipitating from the cell extract ammonium sulfate together with the peptide $R32NS1_{81}$, forming a suspension of said precipitate and separating therefrom the supernatant containing the peptide $R32NS1_{81}$;

d) adjusting said supernatant liquid to a pH of about 2 with acid, thereby precipitating bacterial contaminants, and separating the precipitated bacterial contaminants from the supernatant containing $R32NS1_{81}$;

e) adding a chaotropic agent to the supernatant containing $R32NS1_{81}$ and contacting the supernatant with a cation exchanger, followed by elution at a pH of about 6.5 and collecting the eluate;

f) removing residual bacterial contaminants from said ion exchange eluate by reversed-phase high performance liquid chromatography (HPLC), using as the stationary phase C2-18 alkyl groups on a solid support, and as the mobile phase, a polar organic solvent to provide an eluate which is free of bacterial contaminants; and

g) subjecting the reversed-phase HPLC eluate to diafiltration to yield a retentate of pure polypeptide $R32NS1_{81}$.

In another preferred embodiment, the invention is a process for isolating and purifying the polypeptide $R32tet_{32}$ from a cell culture of E. coli producing said peptide, which process comprises:

a) disrupting the cells and separating the cellular debris from said suspension to provide a clarified cell extract containing the peptide $R32tet_{32}$ together with undesired polypeptides, proteins, DNA and endotoxins;

b) heating the clarified extract to a temperature of about 75°C to about 90°C, so as to precipitate selectively undesired bacterial contaminants, without substantial precipitation or degradation of the polypeptide $R32tet_{32}$, and thereafter cooling the cell extract and separating the precipitated bacterial contaminants from the supernatant containing R32tet$_{32}$;

c) adding ammonium sulfate to the cooled supernatant containing R32tet$_{32}$ to a concentration of about 25% to about 40% of saturation, precipitating from the supernatant ammonium sulfate together with the peptide R32tet$_{32}$, forming a suspension of said precipitate and separating therefrom the supernatant containing the peptide R32tet$_{32}$;

d) adding to the supernatant liquid a soluble salt so as to increase the ionic strength of said supernatant liquid and adjusting said supernatant liquid to a pH of about 2 with acid, thereby precipitating bacterial contaminants and separating the precipated bacterial contaminants from the supernatant containing R32tet$_{32}$;

e) subjecting the supernatant of the acid precipitation to diafiltration using an ammonium acetate buffer containing dithiothreitol at a pH of about 5; thereby yielding a retentate containing peptide R32tet$_{32}$;

f) contacting the retentate with a cation exchanger, followed by elution with a salt at a pH of about 5, and collecting the eluate;

g) removing residual bacterial contaminants from said ion exchange eluate by reversed-phase high performance liquid chromatography (HPLC), using as the stationary phase C2-18 alkyl groups on a solid support, and as the mobile phase, a polar organic solvent to provide an eluate free of bacterial contaminants; and

h) subjecting the reversed-phase HPLC eluate to diafiltration to yield a retentate of pure polypeptide R32tet$_{32}$.

In a third preferred embodiment, the invention is a process for isolating and purifying the polypeptide R32LA from a cell culture of <u>E. coli</u> producing said peptide, which process comprises:

a) disrupting the cells and separating the cellular debris from said suspension to provide a clarified cell extract containing the polypeptide R32LAtogether with undesired polypeptides, proteins, DNA and endotoxins;

b) heating the clarified extract to a temperature of about 75°C to about 90°C, so as to precipitate selectively undesired bacterial contaminants, without substantial precipitation or degradation of the polypeptide R32LA, and thereafter cooling the cell extract and separating the precipitated bacterial contaminants from the supernatant containing R32LA;

c) adding ammonium sulfate to the cooled supernatant containing R32LA to a concentration of about 25% to about 60% of saturation, precipitating from the supernatant ammonium sulfate together with the peptide R32LA, forming a suspension of said precipitate and separating therefrom the supernatant containing the peptide R32LA;

d) adjusting the supernatant liquid containing the peptide R32LA to a pH of about 2 with acid, thereby precipitating bacterial contaminants and separating the precipitated bacterial contaminants from the supernatant containing R32LA;

e) adjusting the supernatant to a pH of about 6.5 and contacting the supernatant with an anion exchanger and collecting the eluate;

f) removing residual bacterial contaminants from said ion exchange eluate by reversed-phase high performance liquid chromatography (HPLC), using as the stationary phase C2-18 alkyl groups on a solid support and, as the mobile phase, a polar organic solvent to provide an eluate free of bacterial contaminants; and

g) subjecting the reversed-phase HPLC eluate to diafiltration to yield a retentate of pure polypeptide R32LA.

The preferred embodiment of the invention yields polypeptide derived from P. falciparum CS protein, such as $R32tet_{32}$, $R32NS1_{81}$ or R32LA, which is pure, that is, contains no measurable undesired polypeptides or proteins, 2 ng/mg or less of DNA and less than 10 Endotoxin Units (EU)/mg. As used herein, the term "Endotoxin Unit" refers to the activity in a defined weight of the U.S. Standard Endotoxin. By definition, 1.0 EU is equal to 0.2 ng of U.S. Standard Endotoxin, lot EC-2. The Office of Biologics (U.S.F.D.A.) establishes this standard and maintains continuity of the EU with successive lots of the U.S. Standard Endotoxin. Such pure polypeptide causes no adverse effects in a patient due to contaminants upon administration in an amount which is effective to produce the desired immune response.

## DETAILED DESCRIPTION OF THE INVENTION

Immunogenic polypeptides comprising the tetrapeptide repeats of the P. falciparum circumsporozoite protein, such as those disclosed in EP-A-192,626 and EP-A-191,748, referred to above, can be purified according to the process of the present invention. These include Rtet32 polypeptides, Rtet86 polypeptides, RG polypeptides, RLR polypeptides, NS1R polypeptides, RNS1 polypeptides, $RNS1_{81}$ polypeptides and RN polypeptides. This process is especially useful for the preparation of malaria vaccine from $R32tet_{32}$, $R32NS1_{81}$ and R32LA, which have been found to be highly effective for causing immune response in mammals to P. falciparum sporozoites. R32tet and R32LA are described in EP-A-192,626 (U.S.S.N. 699,116) and EP-A-191,748 (U.S.S.N. 699,115), cited above.

R32NS1$_{81}$ comprises the same R32 antigenic sequence fused to 80 N-terminal amino acids of NS1. In the fusion, R32 is fused to the second amino acid of NS1; at the C-terminus, amino acid 81 of NS1 is fused to a sequence of -liu-val-asn. Thus, the sequence is:

N-asp-pro (asn-ala-asn-pro)$_{15}$-(asn-val-asp-pro)$_{1}$-(asn-ala-asn-pro)$_{15}$-asn-val-NS1$_{81}$-C

wherein the NS1$_{81}$ sequence is

-asp-pro-***-met-leu-val-asn-C.

This polypeptide is prepared by use of the techniques disclosed in the above-cited patent documents.

As noted above, the process of the present invention involves subjecting a clarified cell lysate, derived from a recombinant E. coli host cell culture and containing pyrogenic, proteinaceous and nucleic acid contaminants of cellular origin, to a series of precipitation and chromatographic procedures including reversed-phase high performance liquid chromatography (HPLC) to yield the desired immunogenic polypeptide substantially free of contaminants.

The recombinant E. coli host is prepared by standard recombinant DNA techniques. See, for example, Young et al. Science, 228: 958 (1985), which is incorporated herein by reference. Such recombinant cells are cultured in nutrient media containing assimilable sources of carbon, nitrogen and minerals, in a presence of oxygen, by standard fermentation techniques. Following fermentation for a time sufficient to express the immunogenic CS polypeptide, cells are collected, such as by centrifugation or filtration. The resulting cell paste is then resuspended and subjected to lysis.

Cell lysis can be accomplished by addition of lysozyme or other lysing or permeabilizing agent to a buffered suspension of the cell pellet at a cell concentration of about 100-300 g/L, based on the wet cell pellet weight. The weight of the cell pellet in production scale operation may range from 800-3,000 g, depending on the particular polypeptide undergoing purification. A suitable lysis buffer is Tris (50 mM), EDTA (2 mM), dithiothreitol (DDT) (0.1 mM), and glycerol (5%) having a pH of 8.0. Alternatively, a buffer comprising sodium phosphate (50 mM), EDTA (2 mM) and glycerol (5%), having a pH of 6.5 may be used. Lysis may also be performed by mechanical or ultrasonic disruption means in an absence of lysozyme. Satisfactory results have been obtained on a production scale using a glass bead Dynomill (Impandex, Maywood, NJ) or a Gaulin homogenizer (APV Gaulin, Inc., Everett, Massachusetts). A combination of chemical, mechanical and/or ultrasonic lysing means may be employed, if desired.

The lysed suspension is can be treated with a detergent, such as deoxycholate, e.g. sodium salt, (approx. 0.1%), to prevent binding of the desired immunogenic polypeptide to the cell debris, although this procedure is not necessary. The deoxycholate may be incorporated lysis buffer, if desired. The lysed suspension is clarified, e.g., by continuous centrifugation at 39,900 x g in a Beckman JCF-Z rotor at a flow rate of 100-500 ml/min.

The clarified extract is then treated to selectively precipitate bacterial contaminants, i.e., proteins and, preferably, also nucleic acids. Such precipitation can be carried out by a variety of means. For example, the $Rtet_{32}$, Rtet86, RLA, RG, RN, RNS1, $RNS1_{81}$ and NS1R polypeptides are heat stable and soluble at about 80°C. Therefore, bacterial protein contaminants are conveniently selectively precipitated by heating the clarified cell lysate to approximately 75-90°C, preferably about 80°C. By way of further example, chemical agents which selectively precipitate nucleic acids can be employed. Polyethyleneimine (PEI), about 0.1 to 1%, for example, removes most of the E. coli DNA and RNA and some of the soluble bacterial proteins. In one embodiment of the invention, the clarified cell extract is treated with PEI by addition of the PEI following disruption. Other selective precipitation procedures which can be employed include salting out such as with sulfate, phosphate or citrate salts with monovalent cations such as ammonium, potassium or sodium; precipitation with organic solvents such as a C 1-3 alcohol, acetone, acetonitrile or tetrahydrofuran; precipitation with organic polymers such as polyethylene glycol or with charged polyelectrolytes such as polyacrylates, caprylic acid salts and rivanol; and precipitation by pH adjustment. By such selective precipitation steps, most of the malaria antigen, e.g., greater than 75%, remains in solution.

Because the P. falciparum repeat region is largely stable at high temperature, heat precipitation is especially useful in purification of peptides not having less heat-stable sequences fused thereto, e.g., the RLA, RG and RN polypeptides. Polyethyleneimine precipitation, however, was not very effective as an initial purification step in the case of another polypeptide fused to the $tet_{32}$ sequence.

Following the initial step, the malaria antigen is selectively precipitated. The preferred technique for selectively precipitating the malaria antigen is salting out, as described above, preferably using ammonium sulfate.

Ammonium sulfate is admixed with the supernatant from the first selective precipitation to a concentration sufficient for selective precipitation of the polypeptide

derived from P. falciparum CS protein. In the case of R32tet$_{32}$, the concentration of ammonium sulfate should be between 25% and 40% of saturation; in the case of R32LA addition of ammonium sulfate between 25% and 60% of saturation is useful for selective precipitation of the desired polypeptide; in the case of the NS1 construct, ammonium sulfate at 20-40% of saturation is useful. The ammonium sulfate addition may be carried out in stages, whereby those proteins precipitable by the ammonium sulfate saturation at each selected stage are removed. The ammonium sulfate is preferably added over a 60 minute period at 4°C, with stirring for an additional 30 minutes at 4°C. The suspension is then centrifuged to yield a pellet containing the crude immunogenic polypeptide. As a result of this selective precipitation, substantially all of the immunogenic polypeptide is contained in the pellet. The ammonium sulfate pellet is then redissolved in a suitable buffer.

The redissolved polypeptide is then subjected to a third selective precipitation designed to remove bacterial nucleic acids. This step is preferably carried out by acidification. In practice, for the R32tet$_{32}$, the ionic strength of the partially purified cell lysate is increased significantly, in conjunction with the acid treatment, by the addition of a salt having high solubility in the cell lysate. Suitable salts for this purpose include 2-4 M MgCl$_2$ or 2-4 M NaCl, or mixtures thereof. Thereafter, the cell lysate is adjusted to a pH of about 2.0 with a suitable acid, such as trifluoroacetic acid, phosphoric acid or hydrochloric acid. Nucleic acids precipitated by the acid treatment can be easily removed from the cell lysate by centrifugation.

Partial purification of the cell lysate, in the manner just described, significantly reduces the amount of cellular polypeptides and proteins initially present in the cell lysate. Following the selective precipitation steps, the solution containing the immunogenic polypeptide is further purified by a series of two chromatographic procedures, namely, ion exchange chromatography and reversed-phase chromatography.

Separation of residual bacterial contaminants from the partially purified cell lysate by ion exchange is carried out using a microparticulate column packing having cation or anion exchange groups, such as carboxymethyl (CM), sulfopropyl (SP), diethylaminoethyl (DEAE), quarternary aminoethyl (QAE), bound to a suitable matrix. The ion exchanger should provide a sufficiently porous, open matrix for passage of the polypeptide to be purified. Generally, an ion exchanger having a bead size from 10-100 microns in diameter, with an exclusion limit of 10 daltons will perform satisfactorily. Particularly good results have been obtained using a CM cation exchange support such as CM-Trisacryl$^R$M (LKB Products, Bromma, Sweden) for ion exchange of $R32tet_{32}$. The highly basic C-terminal "tail" binds tightly to the CM-support, effecting separation of the desired polypeptide from other contaminants then present in the cell lysate. Separation of nucleic acids from a partially purified cell lysate containing the polypeptide R32LA has been accomplished on the anion exchange support, DEAE-Trisacryl M (LKB Products, Bromma, Sweden). In the case of $R32NS1_{81}$, particularly good results have been obtained using the cation exchange support, sulfopropyl-Sepharose$^R$ (Pharmacia, Piscataway, New Jersey) cross-linked agarose.

The solution of the immunogenic peptide is contacted with the ion exchange support and then eluted therefrom. Elution can be carried out using a suitable buffer solution which provides a fraction containing the desired immunogenic polypeptide, substantially free of polypeptide, protein and nucleic acid contaminants. The buffer solutions used as eluants herein are those widely used in ion exchange chromatography of biological substances. Gradient elution from a cation exchange support is used to advantage with certain of the polypeptides derived from P. falciparum CS protein containing the tet "tail". For the polypeptide R32LA, the nucleic acid contaminants are adsorbed on the ion exchange matrix, whereas the desired polypeptide passes through the ion exchange and is collected in the eluate and wash. This effluent may be passed several times in succession through the same ion exchange column, or through separate columns having different packings.

In the case of $R32NS1_{81}$, it is preferred that a chaotrope, e.g., urea, thiocyanate, guanidine, guanidinum chloride or ethylene glycol, is added to the supernatant containing the peptide from the salt precipitation and the supernatant is adjusted to about pH 4 with, for example, sodium hydroxide, prior to ion exchange. The preferred chaotrope is urea at a final concentration of about 3M. The chaotrope and pH adjustment is useful in disrupting aggregates of the peptide. Following adsorption to a cation exchange support, the support is washed with buffer at pH 4 and at pH 5, e.g., 50 mM sodium acetate. Then, the $R32NS1_{81}$ is eluted with salt at about pH 6.5, e.g., 20 mM sodium phosphate and 0.5 M sodium chloride in 10 mM DTT.

The cell lysate, after treatment for removal of polypeptide, protein and nucleic acid contaminants, as described above, is rendered substantially free of residual bacterial contaminants including pyrogenic material, such as cellular endotoxins, and also including residual other contaminants by reversed-phase HPLC.

For example, reversed-phase HPLC was important for resolution of closely related antigens in the product mixture. A good example of component resolution is the purification of R32LA. In this case, even though the major species in the mixture is in excess of 95%, separation of 2 proteins differing by 3 amino acid residues is achieved. Similar separations during preparation of other proteins where truncated or oxidized C-terminal derivatives were removed by reversed-phase HPLC were achieved.

Reversed-phase chromatography involves contacting a solution of a desired protein, a solid, hydrophobic support, or stationary phase, whereby the protein is adsorbed to the support. The protein is then eluted, after washing, by rinsing the support with an a polar organic solvent, i.e., the mobile phase. The stationary phase preferably comprises a support such as alumina, or a silica-based support, the latter being preferred, to which is bonded various non-polar organic groups. Such bonded phases may be prepared, for example, by reacting surface silanol groups on the silica with an organochlorosilane, as is well-known in the art. The silica-based supports include, for example, spherical silica particles, irregular silica particles or particulate substrates coated with silica. The particle size and porosity must be appropriate for separation of the specific polypeptide which is to be purified. Separation of contaminants from the immunogenic polypeptides by reversed-phase HPLC is preferably carried out using a stationary phase selected from the group of C2-C18 alkyl groups, preferably, on a silica-based support.

The mobile phase chosen for reversed-phase HPLC should have low toxicity and viscosity and be readily available in pure form. The mobile phase may be selected from the group of water miscible lower alcohols, e.g. methanol, n-propanol, or isopropanol, tetrahydrofuran, dioxane, or acetonitrile. The preferred mobile phase for use in the present invention is selected from the group of $C_1$-$C_3$ alcohols, acetonitrile or tetrahydrofuran. The mobile phase chosen will depend primarily on the strength and selectivity of a given solvent for the immunogenic polypeptide sought to be purified.

Gradient elution, e.g. using 0-35% isopropanol in acid, e.g., heptafluorobutyric acid, phosphoric acid, acetic acid or trifluoroacetic acid, has been found to be effective in the purification of $R32NS1_{81}$, $R32tet_{32}$ and R32LA. Triflouroacetic acid, 0.1 to 0.2% by volume is preferred.

Under optimal conditions, as exemplified below, reversed-phase HPLC is capable of achieving a $10^8$ reduction of the endotoxin content of a partially purified cell lysate in a single step. Diafiltration and sterile filtration are also performed on the eluate of the reversed-phase HPLC column as a final purification step for removal of acids and organic solvents used in HPLC and to adjust pH to pH 6-8.

Various other procedures can be employed in connection with the process of the present invention, although such other procedures are not necessary to achieve a highly purified, pharmaceutical grade product. Such procedures can be employed between, before or after the above described process steps. One such optimal step is diafiltration.

The term "diafiltration" is used herein in the art-recognized sense, to refer to a form of continuous dialysis which is extremely effective in achieving many buffer exchanges. Diafiltration is preferably carried out across a cellulosic membrane or ultrafilter. Suitable membranes/filters are those having from about a 1000 molecular weight (MW) cut-off to those having pore size up to 2.4 um diameter. A number of different systems adaptable to diafiltration are commercially available, such as the 10K Amicon dual spiral cartridge system. In the process of the present invention, diafiltration using an ammonium acetate buffer containing DTT at about pH 5 can be effectively employed in the purification of the polypeptide R32tet$_{32}$, prior to ion exchange, in order to remove low molecular weight contaminants. Following the purification, the solution containing the pure poplypeptide can be diafiltered to remove residual salts.

Another procedure which has been discovered to be useful for disruption of protein-polynucleotide complexes and, therefore, for removal of nucleic acid contaminants, is treatment of the impure antigen with nucleases. Nuclease digestion can be incorporated in the process of the invention immediately following cell lysis or following partial purification, such as after heating and/or after ammonium sulfate precipitation. For example, the ammonium sulfate isolate can be diafiltered, as described above, except that the buffer is selected for its compatibility with nuclease enzyme activity and the antigen. A suitable buffer for this purpose is 10 mM ammonium acetate and 20 mM $ZnSO_4$ (pH 5.0). Following the diafiltration, a nuclease enzyme is added to the retentate containing the protein in the diafiltration apparatus. After a holding period of sufficient duration to permit hydrolysis of nucleic acid to low molecular weight nucleotides, diafiltration is continued using a high salt buffer to weaken ionic interactions between the nucleotide fragments and the antigen. During this phase, low molecular weight nucleotide fragments are removed in the permeate while the antigen is retained in the retentate. The diafiltration concludes using a third buffer which is the same used in the original procedure.

Any enzyme or combination of enzymes having phosphodiesterase activity with nucleic acid substrates in buffers compatible with the antigen can be used. Phosphodiesterases called nucleases which hydrolyze single and double stranded DNA and RNA, and act by endo- and exohydrolytic mechanisms are preferred. $P_1$ nuclease produced by _Penicillium citrinum_ and Micrococcal nuclease are examples of preferred nucleases.

Size exclusion chromatography may be used as an adjunct to reversed-phase HPLC in the practice of the present invention.

Size exclusion chromatography, when used, can be conveniently performed using a porous particulate matrix having a working range of 1000-300,000 daltons and particle size between about 10 and about 100 microns in diameter. Suitable size exclusion column packings for use in the present invention include Spherogel$^R$ TSK 2000 SW and 3000 SW, (Beckman Instruments, Berkeley, CA) which are spherical silica particles with a protein-compatible hydrophilic polymer coating and an average particle size of 13 microns. Other commercially available size exclusion materials may also be used, such as Sephadex $^R$G-50, G-75, G-100 or Sephacryl$^R$ S-200 (Pharmacia, Piscataway, NJ) or Biogel$^R$ P-10 to P-60 (BioRad, Richmond, CA).

Thus, purification of R32tet$_{32}$ by the process of the invention preferably comprises the following steps: (1) lysis (2) heat treatment (3) ammonium sulfate precipitation (4) acid precipitation (5) diafiltration (6) ion exchange (7) reversed-phase HPLC (8) diafiltration and (9) sterile filtration. The purification of R32LA is preferably carried out by the following steps: (1) lysis (2) heat treatment (3) ammonium sulfate precipitation (4) acid precipitation (5) ion exchange (6) reversed-phase HPLC (7) diafiltration and (8) sterile filtration. The purification of R32NS1$_{81}$ is preferably carried out by the following steps: (1) lysis (2) PEI precipitation (3) ammonium sulfate precipitation, (4) acid precipitation, (5) ion exchange, (6) reversed-phase HPLC, (7) diafiltration and (8) sterile filtration.

The following examples are, illustrative, but not limiting, of the process of the invention. Examples 1 and 2 describe fermentation and cell harvesting of a recombinant E. coli host cell producing R32tet$_{32}$.

Example 1 - Fermentation

A master cell bank was prepared by selecting a kanamycin resistant clone of Escherichia coli K12, strain AR58, containing plasmid pR32tet$_{32}$Kn from the agar plates containing 50 µg/ml kanamycin sulfate (Kn) and incubating at 32°C. Strain AR58 is an I857 lysogen. pR32tet32Kn is a derivative of pAS1, which is described in Rosenberg, U.S. patent 4,578,355. The vector is substantially described in Young et al., Science 228: 958 (1985), except that Kanamycin resistance was substituted for ampicillin resistance in pASi. The master cell bank was frozen at -65°C for storage.

A working cell bank was prepared from the master cell bank and was stored frozen at -65°C.

A seed culture medium was prepared from glycerol (26 gms.), yeast extract (24 gms.), tryptone (12 gms.) K$_2$HPO$_4$ (15.3 gms.), KH$_2$PO$_4$ (1.7 gms.), (NH$_4$)$_2$SO$_4$ (2.0), PPG 2000 (0.5 ml.) kanamycin (shake flask only; 50.0 µg/ml.), and sufficient deionized water to bring the volume to one liter. The pH of the seed culture medium was 7.1-7.2.

A vial from a working cell bank was removed from liquid nitrogen storage and thawed at room temperature. An aliquot of the thawed suspension was transferred to each of two shake flasks containing sterile seed medium to which sterile Kn had been added. The shake flasks were incubated on a gyratory shaker for approximately 15 hours at 32°C. A sample of the culture was removed from each shake flask and the optical density measured, as described in Example 4 below. The value obtained was used to calculate the volume of seed culture required to provide a specific optical density (greater than 0.1) in the fermentation medium after inoculation. The calculated volume of the inoculum was transferred to a sterile aspirator flask.

The fermenter containing incomplete culture medium, made up of the same components as the seed culture medium, but omitting the potassium phosphate salts, was sterilized in situ at 121°C with agitation for 15 minutes. A solution of the potassium phosphate salts was sterilized separately by autoclaving. The sterile solution was added aseptically to the sterile incomplete culture medium in the fermenter. The resultant composition of the complete medium was that described above for the seed culture medium.

Inoculation of the fermenter was accomplished by pumping the inoculum prepared above from the aspirator flask through an addition port under aseptic conditions. During growth, temperature was controlled at 32°C, pH was controlled above 6.5 by the addition of sterile $NH_4OH$, and the dissolved oxygen was controlled above 15% of saturation.

Samples were removed during the growth phase to determine the optical density. When the appropriate density (greater than OD 12.0) had been reached, expression of the antigen was induced by raising the temperature of the culture from 32°C to 42°C. (See, Rosenberg et al., Meth. Enzymol. 101: 123 (1983). The fermentation was continued under these conditions for 90-240 minutes.

Example 2 - Cell Harvesting

The broth culture was chilled below 20°C and transferred from the fermenter to a hollow fiber concentrator, Amicon DC10L, equipped with a 0.1 micron cartridge. When the transfer was complete the concentrator was placed in a cold room at 4°C. The filtration proceeded until the retentate volume was approximately 15% of the culture volume. After the filtration was completed, the retentate was drained into an aspirator flask. The aspirator flask was disconnected and placed in a Class II, type B biological hood. The aspirator contents were dispensed into bottles and the suspension was centrifuged. The supernatant was removed and the pellets divided among several containers. The cell pellets were stored at -70°C until initiation of the purification process.

Examples 3-9 describe an isolation procedure and various purification procedures for the recovery of purified polypeptide R32tet$_{32}$.

Example 3 - Isolation of R32tet$_{32}$ From
Producing Cells

A cell pellet, prepared as described in Example 2, above, and weighing 20 g, was thawed by suspension, at room temperature, in a lysis buffer made up of 50 mM Tris-HCl, 2 mM EDTA, 0.1 mM DTT, 5% glycerol (pH 8) to a concentration of approximately 1 g/5 ml, based on the wet cell pellet weight. All subsequent steps were performed at 4°C unless otherwise indicated.

Lysozyme was added to the cell suspension to a final concentration of 0.2 mg/ml and the suspension was stirred for 30 minutes. The solution was blended three times for intervals of one minute each in a Waring blender and then sonicated three times for intervals of one minute each using a Branson Model 350 sonifier set at 40% duty cycle and with an output value of 6. Deoxycholate (DOC) was added to the lysed suspension to a concentration of 0.1% (w/v). The mixture was stirred for 30 min. and then centrifuged at 10,000 x g in a Sorvall Model RC-5B centrifuge for 60 minutes.

The supernatant obtained by centrifugation was heated in a boiling water bath for 10 minutes with stirring and then cooled for 1 hour at room temperature. The heat treated suspension was centrifuged as indicated above for 30 minutes. Granular ammonium sulfate was added gradually with stirring to the heat treated supernatant to a concentration of 15-25% of saturation over a 15 minute interval and the solution was stirred for 30 minutes. The suspension was again centrifuged as indicated above for 30 minutes to yield a pellet containing crude R32tet$_{32}$. The pellet was resuspended in one fifth volume of lysis buffer.

Example 4 - Purification of R32tet$_{32}$ By Acid Precipitation, Ion Exchange Chromatography and Reversed-Phase HPLC

While stirring at 4°C, the solution resulting from Example 3 was adjusted to 1-2 M magnesium chloride. The pH was adjusted to 2 with trifluoroacetic acid and the solution stirred for several hours. The precipitated nucleic acids were removed by centrifugation for 30 minutes.

The acid-treated supernatant was chromatographed on CM-Trisacryl M. The supernatant was pumped onto a column of CM-Trisacryl M (1-8 mg protein loaded/ml of gel) which was previously equilibrated with 10 mM ammonium acetate (pH 5), at a flow rate of 60 cm/hr. The column was washed with 10 mM ammonium acetate (pH 5). The product was eluted with either a linear gradient of 0-0.5 M ammonium chloride or a step gradient of 0.3 and then 0.6 M ammonium chloride. The desired product eluted at 0.5 M salt in the linear gradient and 0.6 salt in the step gradient as determined by monitoring at 280 nm.

The eluate from the ion exchange column was dialyzed against 10 mM phosphate buffer (pH 6.5) and made 10 mM in DTT. The dialyzed solution was adjusted to 5% acetic acid and chromatographed on a Vydac 300 Angstrom C-4, 5 micron reversed-phase column (0.46 X 25 cm) using a gradient of 2-propanol in 5% acetic acid. The desired product eluted at a solvent concentration of 30% as determined by monitoring at 229 nm. The desired product was dialyzed against pyrogen-free 10 mM phosphate, 0.15 M chloride buffer (pH 6.5). This procedure resulted in R32tet$_{32}$ having about 6 Endotoxin Units per mg of protein and in the order of 0.001% (W/W) DNA.

Example 5 - Nuclease Treatment

An ammonium sulfate precipitate of the polypeptide R32tet$_{32}$, prepared as described above, was redissolved in 10 mM Tris, 10 mM DTT (pH8). The solution was diafiltered against 10 volumes of 10 mM ammonium acetate, 20 mM ZnSO$_4$ (pH5) using a spiral ultrafiltration system (Amicon S1Y10) with a nominal 10,000 dalton cutoff. The recirculation pump was stopped and the recirculation vessel containing the retentate was opened. $P_1$ nuclease, a zinc requiring enzyme, was added to the recirculation vessel to a concentration of 10 mg/l. The retentate was incubated for 1 hour at 37°C with stirring. Diafiltration was resumed using 10 volumes of 2M $MgCl_2$ followed by ten volumes of 10 mM $NH_4OAC$ (pH5). The retentate was subsequently purified by the same ion exchange (CM-) and HPLC-chromatography procedures described above.

The effectiveness of the nuclease diafiltration procedure was compared with the standard diafiltration procedure using an ammonium sulfate isolate which typically contains 100,000 ng DNA/mg protein. The nuclease diafiltration procedure yields a product containing 100 ng DNA/mg protein, compared with 14,000 ng DNA/mg protein in the product from the standard diafiltration procedure. This represents a 1000-fold reduction in nucleic acid as a result of the nuclease diafiltration step and 140-fold reduction in contaminating DNA over the standard diafiltration process.

Examples 6 and 7 describe production scale isolation and purification protocols for the polypeptides $R32tet_{32}$ and R32LA, respectively.

Example 6 - Production Scale Isolation and
Purification Protocol for the
Polypeptide $R32tet_{32}$

A cell pellet, prepared as described in Example 2, above, and weighing 2600 g. was thawed to room temperature and suspended in a buffer made up of 50 mM Tris, 2 mM EDTA, 0.1 mM DTT, 5% glycerol (pH 8.0) to a concentration of approximately 200 g/l, based on wet cell pellet weight. Lysozyme was added to a final concentration of 0.15 mg/ml and the mixture was incubated for 30 minutes at 4°C.  This suspension was pumped through a Dynomill glass bead cell disrupter, using 0.2 mm beads at a rate of 100 ml/min. with cooling to 4°C.  The lysed suspension was treated with 0.1% deoxycholate for 30 minutes at 4°C followed by continuous centrifugation at 39,9000 X g in a Beckman JCF-Z rotor, at a flow rate of 250 ml/min.

The supernatant obtained by centrifugation was diluted to approximately 150 g/l and heated on a steam bath to approximately 80°C to 90°C and then cooled to 15-35°C.  This heat treated suspension was centrifuged, as described above.

Granular ammonium sulfate was added over a 30 minute period to 25% saturation, centrifuged and the precipitate was removed.  To the supernatant liquid, granular ammonium sulfate was added gradually with stirring to a concentration of approximately 40% of saturation, over a 30 minute period at 4°C.  The suspension was centrifuged as described above, to yield a pellet containing the crude polypeptide R32tet$_{32}$.  The ammonium sulfate pellet was suspended in one fifth volume of 10 mM Tris buffer containing 10 mM DTT, stirred overnight at 4°C and centrifuged at 14,000 X g at 4°C for 30 minutes.

The supernatant from the redissolved pellet was adjusted to 2 M in MgCl$_2$ and adjusted to pH 2 with trifluoroacetic acid, and stirred at 4°C for two hours. The precipitated nucleic acids were removed by centrifugation at 14,000 X g at 4°C for 30 min.

The supernatant from the acid precipitation was diafiltered with 10 volumes of 10 mM pH 5.0 ammonium acetate buffer containing 10 mM DTT using a 10 K Amicon dual spiral cartridge system.

The diafiltered retentate was pumped onto a 1.2 liter column of CM-Trisacryl M at 100 ml/min and the column was eluted with a step gradient of ammonium chloride (0, 0.3 and 0.6 M) in 10 mM pH 5 ammonium acetate buffer. The desired product eluted at 0.6 M ammonium chloride.

The ion-exchange product was made 10 mM in DTT and 5% in acetic acid and chromatographed on Vydac 300 Angstrom C-4 15 to 20 micron reversed-phase packing in a 5.1 X 30 cm column with a gradient of isopropanol in 5% acetic acid using a Rainin Autoprep HPLC unit at 100 ml/min. The product eluted at approximately 18% isopropanol as determined by monitoring at 280 nm.

The reversed-phase product was diafiltered with 7 l. of sterile pyrogen-free PBS buffer (10 mM pH 6.6 phosphate, 150 mM sodium chloride, filtered through a 10 K hollow fiber cartridge) using a 10 K Amicon spiral cartridge to yield sterile bulk product, after sterile filtration.

The results of a typical production run using the above-described procedure are set forth in Table II, below.

TABLE II

ANALYSIS OF PRODUCTION RUN

R32tet$_{32}$ ANTIGEN

| Step | Total[a] Protein (g) | Antigen[b] (g) | Endotoxin[c] LOG EU/mg | DNA[d] ng/mg |
|---|---|---|---|---|
| Lysate | 368.0 | 32.0 | 10.0 | 600000 |
| Heated supernatant | 116.0 | 10.0 | 10.0 | 1400000 |
| Ammonium sulfate pellet | 11.0 | 3.4 | 6.0 | 90000 |
| Acid supernatant | 6.0 | 3.5 | 4.5 | <0.5 |
| Diafiltrate | 3.3 | 2.5 | 5.5 | <20 |
| Ion Exchange | 1.5 | 2.0 | 2.6 | <0.5 |
| Reversed-Phase | 1.3 | 1.9 | 0.5 | <0.5 |
| Final Product | 1.0 | 1.3 | 0.8 | 0.5 |

a - Determined by Lowry analysis using albumin as a standard; lower levels of total protein than antigen at later stages of protocol attributed to lower Lowry response of antigen.

b - antigen levels estimated using particle fluorescense assay with correction by HPLC analysis.

c - Determined by Limulus Amebocyte Lysate clotting assay and expressed as log of total EU (endotoxin units) per mg of antigen.

d - Determined at early states of protocol by diphenylamine colormetric test and at later stages by hybridization, and expressed in terms of ng DNA per mg of antigen.

Example 7 - Production Scale Isolation and
               Purification Protocol for the
               Polypeptide R32LA

A cell pellet obtained from a cell culture of E. coli producing R32LA (the sequence of which is:  N-Met-Asp-Pro [Asn-Ala-Asn-Pro)$_{15}$ - (Asn-Val-Asp-Pro)]$_2$ - Leu Arg-C), and weighing 1000 g, was thawed at room temperature and suspended in a buffer made up of 50 mM phosphate, 2 mM EDTA, 5% glycerol containing 0.1% deoxycholate (pH 6.5) to a concentration of approximately 200 g/l, based on wet cell pellet weight.  The suspension was pumped through a Dynomill glass bead cell disrupter using 0.2 mm beads at a rate of 100 ml/min. with cooling to 4°C.  The lysed suspension was clarified by continuous centrifugation at 39,900 X g in a Beckman JCF-Z rotor at a flow rate of 300 ml/min.

The supernatant obtained by centrifugation was heated on a steam bath to approximately 80°C and then cooled to about 15°C. The heat treated suspension was centrifuged as described above.

Granular ammonium sulfate was added gradually with stirring to the heat-treated supernatant to a concentration of 25% of saturation over a 30 minute period at 4°C. The suspension was centrifuged at 39,900 X g at 200 ml/min in the Beckman JCF-Z rotor to yield a supernatant containing the crude polypeptide R32LA. Granular ammonium sulfate was added gradually with stirring to the heat-treated supernatant to a concentration of 60% of saturation over a 30 minute period at 4°C and the solution was stirred an additional 15 minutes at 4°C. The suspension was again centrifuged under the same conditions to yield a pellet containing the crude polypeptide R32LA. The ammonium sulfate pellet was resuspended in one fifth volume of 10 mM pH 6.5 phosphate buffer, stirred for one hour at 4°C and centrifuged at 14,000 X g at 4°C for 30 minutes.

The supernatant from the redissolved pellet was adjusted to pH 2 with trifluoroacetic acid, and stirred overnight at 4°C. The precipitated nucleic acids were removed by centrifugation at 14,000 X g at 4°C for 30 minutes. The supernatant from the acid precipitation was neutralized to pH 6.5 by dropwise addition of 6 M ammonium hydroxide with stirring at 4°C.

The neutralized supernatant was pumped on to a 2.0 liter column of DEAE-Trisacryl M at 100 ml/min and the column is washed with a 10 mM phosphate containing 0.1 M sodium chloride (pH 6.5). The eluate and wash contained the polypeptide R32LA. The column was regenerated with 10 mM phosphate buffer containing 1 M NaCl (pH 6.5). The first wash containing the polypeptide R32LA was passed through the column a second time followed by a wash with

10 mM phosphate containing 0.1 M NaCl (pH 6.5).

The eluate and wash from the ion exchange column was made 0.2% in trifluoroacetic acid and chromatographed on Vydac 300 Angstrom C-18 15 to 20 micron reversed-phase packing in a 5.1 X 30 cm column using a gradient of isopropanol in 0.2% trifluoroacetic acid using a Rainin Autoprep HPLC at 100 ml/min. The product eluted at approximately 10% isopropanol as determined by monitoring at 220 nm.

The reversed-phase product was concentrated to 500 ml and diafiltered with 5 1 of sterile pyrogen-free buffer (10 mM pH 6.6 phosphate, 150 mM sodium chloride, filtered through a 10 K hollow fiber cartridge) using a Lab-20 ultrafiltration system with two 5 K cellulosic membranes. The retentate was filtered thorough a Millipak 60 cartridge to yield sterile bulk product.

The results of a typical production run using the procedure just described are set forth in Table III, below.

## TABLE III

### ANALYSIS OF PRODUCTION RUN R32LA ANTIGEN

| Step | Total[a] Protein (g) | Antigen[b] (g) | Endotoxin[c] LOG EU/mg | DNA[d] ng/mg |
|------|------|------|------|------|
| Lysate | 144.0 | 6.2 | 10 | 1800000 |
| Heated supernatant | 23.0 | 4.2 | 7 | 1400000 |
| Ammonium sulfate | 7.0 | 3.0 | 8 | 700000 |
| Acid supernatant | 3.0 | 3.0 | 4 | 8 |
| Ion Exchange | 3.0 | 2.9 | 2 | <1 |
| Reversed-Phase | 1.9 | 2.6 | 0 | <1 |
| Final Product | 1.6 | 2.0 | 1 | <1 |

a - See Table II, above

b - antigen estimated using HPLC analysis

c - See Table II, above

d - See Table II, above

Example 8 - Purification of R32NS1$_{81}$

R32NS1$_{81}$ is purified substantially as described in Example 6, above, except that a polyethyleneimine (PEI) precipitation step was substituted for the heat treatment. In this step, the clarified cell lysate is incubated in 0.5% PEI while stirring at 2-8$^0$C for about an hour, followed by centrifugation to separate precipitated bacterial nucleic acids and proteins. In the second selective precipitation step, R32NS1$_{81}$ is

collected in ammonium sulfate at 20-40% of saturation. The ammonium sulfate precipitate is resuspended and acid precipitated without addition of chloride salt. Urea is added to the supernatant to a final concentration of about 3 M urea and the pH is adjusted to pH 4 with sodium hydroxide prior to ion exchange.

In a representative ion exchange step, a sulfopropyl-Sepharose column is employed. Such column has a greater capacity than the CM-Trisacryl column used in the preceding Examples because it is less subject to protonation at low pH. The malaria antigen is eluted in 20 mM sodium phosphate, 0.5 M sodium chloride, 10 mM DTT (pH 6.5).

The eluate from the ion exchange step is then treated by reversed-phase chromatography and filtration substantially as described in Example 6, except that 0.2% triflouroacetic acid is used instead of acetic acid, to result in pure $R32NS1_{81}$.

The results of these Examples demonstrate that highly purified potential malaria vaccinal antigens can be produced from $\underline{E.}$ $\underline{coli}$ expression systems. In spite of the unusual amino acid composition of the $\underline{P.}$ $\underline{falciparum}$ CS constructs, each product accumulates to 4-11% of total cellular protein and is stable during and after heat induction. The flexibility of the purification scheme results from the dominating properties of the R32 peptide sequence. These properties (temperature and acid pH stability) result in substantial purification by using only precipitation methods. Although the $tet_{32}$ peptide is less stable to 80°C treatment, the purification factor was so high that protein loss was tolerable for initial production of this material. Thus, the process of the invention can be used to purify and $\underline{E.}$ $\underline{coli}$ derived

immunogenic polypeptide comprising at least 4 tandem repeats from the P. *falciparum* CSP.

The purified immunogenic polypeptide may be formulated as a vaccine by adsorption on or admixing with a suitable adjuvant so as to increase its immunizing potency. Examples of suitable adjuvants include aluminum hydroxide and aluminum sulfate.

While the above fully describes the invention and all preferred embodiments thereof, it is to be appreciated that the invention is not limited to the embodiments particularly described but rather includes all modifications thereof coming within the scope of the following claims.

CLAIMS:

1. A process for purifying an immunogenic polypeptide, comprising four or more tandem repeat units of the *P. falciparum* CS protein, from a clarified cell lysate from a recombinant *E. coli* host cell culture which comprises:

(a) selectively precipitating bacterial contaminants;

(b) selectively precipitating the immunogenic polypeptide from the supernatant of step (a);

(c) resolubilizing the precipitate from step (b) containing the immunogenic polypeptide and selectively precipitating bacterial contaminants from the solution;

(d) contacting the solution of the immunogenic polypeptide with an ion exchange support and collecting fractions which contain the polypeptide; and

(e) contacting the solution of the immunogenic polypeptide with a solid, hydrophobic support whereby the polypeptide is adsorbed to the support, eluting the polypeptide from the support with a polar organic solvent and collecting fractions which contain the purified poplypeptide.

2. The process of claim 1 wherein step (i) is carried out by heating the clarified cell lysate or by precipitation with polyethyleneimine; step (ii) is carried out by salting out the immunogenic polypeptide; step (iii) is carried out by adjusting the pH to below about pH 2.5; and, step (v) is carried out by contacting the solution of the immunogenic polypeptide with a C2-18 alkyl support and eluting the immunogenic polypeptide with a C1-3 alcohol, acetonitrile or tetrahydrofuran.

3. The process of claim 2 wherein step (i) is carried out by heating the clarified cell lysate to 75-90 $^0$C or by precipitation with 0.1-1% polyethyleneimine;

step (ii) is carried out by salting out the immunogenic polypeptide with a sulfate, citrate or phosphate salt with a monovalent cation; step (iii) is carried out by adjusting the pH to pH 2.0-2.4 with triflouroacetic acid, phosphoric acid or hydrochloric acid; step (iv) is carried out using a carboxymethyl or sulfopropyl support; and, step (v) is carried out using a C4 or C18 alkylsilica support.

4. The process of claim 2 wherein step (i) is carried out by heating the clarified cell lysate to 80-90 C or by precipitation with 0.2-1% polyethyleneimine; step (ii) is carried out by salting out the immunogenic polypeptide with a sulfate, citrate or phosphate salt with a monovalent cation; step (iii) is carried out by adjusting the pH to pH 2.0-2.4 with triflouroacetic acid, phosphoric acid or hydrochloric acid; step (iv) is carried out using a DEAE or QAE carboxymethyl or sulfopropyl support; and, step (v) is carried out using a C4 or C18 alkylsilica support.

5. The process of claim 1 wherein the immunogenic polypeptide is selected from the group consisting of Rtet32 polypeptides, Rtet86 polypeptides, RG polypeptides, RLR polypeptides, RN polypeptides, NS1R polypeptides and RNS1 polypeptides.

6. A process for isolating and purifying the polypeptide $R32NS1_{81}$ from a cell culture of E. coli producing said peptide, which process comprises:

a) disrupting the cells and separating the cellular debris from said suspension to provide a clarified cell extract containing the peptide $R32NS1_{81}$ together with undesired polypeptides, proteins, DNA and endotoxins;

b) treating the clarified extract with polyethyleneimine so as to precipitate undesired bacterial contaminants and thereafter separating the precipitated bacterial contaminants from the supernatant containing the peptide $R32NS1_{81}$;

c) adding ammonium sulfate to the supernatant containing $R32NS1_{81}$ to a concentration of about 20% to about 40% of saturation, precipitating from the cell extract ammonium sulfate together with the peptide $R32NS1_{81}$, forming a suspension of said precipitate and separating therefrom the supernatant containing the peptide $R32NS1_{81}$;

d) adjusting said supernatant liquid to a pH of about 2 with acid, thereby precipitating bacterial contaminants, and separating the precipitated bacterial contaminants from the supernatant containing $R32NS1_{81}$; and

e) adding a chaotropic agent to the supernatant containing $R32NS1_{81}$ and contacting the supernatant with a cation exchanger, followed by elution at a pH of about 65 and collecting the eluate; and

f) removing residual bacterial contaminants from said ion exchange eluate by reversed-phase high performance liquid chromatography (HPLC), using as the stationary phase C2-18 alkyl groups on a solid support, and as the mobile phase, a polar organic solvent to provide an eluate which is free of bacterial contaminants; and

g) subjecting the reversed-phase HPLC eluate to diafiltration to yield a retentate of pure polypeptide $R32NS1_{81}$.

7. A process for isolating and purifying the polypeptide $R32tet_{32}$ from a cell culture of _E. coli_ producing said peptide, which process comprises:

a) disrupting the cells and separating the cellular debris from said suspension to provide a clarified cell extract containing the peptide R32tet$_{32}$ together with undesired polypeptides, proteins, DNA and endotoxins;

b) heating the clarified extract to a temperature of about 80°C to about 90°C, so as to precipitate selectively undesired bacterial contaminants, without substantial precipitation or degradation of the polypeptide R32tet$_{32}$, and thereafter cooling the cell extract and separating the precipitated bacterial contaminants from the supernatant containing R32tet$_{32}$;

c) adding ammonium sulfate to the cooled supernatant containing R32tet$_{32}$ to a concentration of about 25% to about 40% of saturation, precipitating from the supernatant ammonium sulfate together with the peptide R32tet$_{32}$, forming a suspension of said precipitate and separating therefrom the supernatant containing the peptide R32tet$_{32}$;

d) adding to the supernatant liquid a soluble salt so as to increase the ionic strength of said supernatant liquid and adjusting said supernatant liquid to a pH of about 2 with acid, thereby precipitating bacterial contaminants and separating the precipated bacterial contaminants from the supernatant containing R32tet$_{32}$;

e) subjecting the supernatant of the acid precipitation to diafiltration at a pH of about 5; thereby yielding a retentate containing peptide R32tet$_{32}$;

f) contacting the retentate with a cation exchanger, followed by elution with a salt at a pH of about 5, and collecting the eluate;

g) removing residual bacterial contaminants from said ion exchange eluate by reversed-phase high performance liquid chromatography (HPLC), using as the stationary phase C2-18 alkyl groups on a solid support, and as the mobile phase, a polar organic solvent to provide an eluate free of bacterial contaminants; and

h) subjecting the reversed-phase HPLC eluate to diafiltration to yield a retentate of pure polypeptide R32tet$_{32}$.

8. A process for isolating and purifying the polypeptide R32LA from a cell culture of E. coli producing said peptide, which process comprises:

a) disrupting the cells and separating the cellular debris from said suspension to provide a clarified cell extract containing the polypeptide R32LA together with undesired polypeptides, proteins, DNA and endotoxins;

b) heating the clarified extract to a temperature of about 75°C to about 90°C, so as to precipitate selectively undesired bacterial contaminants, without substantial precipitation or degradation of the polypeptide R32LA, and thereafter cooling the cell extract and separating the precipitated bacterial contaminants from the supernatant containing R32LA;

c) adding ammonium sulfate to the cooled supernatant containing R32LA to a concentration of about 25% to about 60% of saturation, precipitating from the supernatant ammonium sulfate together with the peptide R32LA, forming a suspension of said precipitate and separating therefrom the supernatant containing the peptide R32LA;

d) adjusting the supernatant liquid containing the peptide R32LA to a pH of about 2 with acid, thereby precipitating bacterial contaminants and separating the precipitated bacterial contaminants from the supernatant containing R32LA;

e) adjusting the supernatant to a pH of about 6.5 and contacting the supernatant with an anion exchanger and collecting the eluate;

f) removing residual bacterial contaminants from said ion exchange eluate by reversed-phase high performance liquid chromatography (HPLC), using as the stationary phase C2-18 alkyl groups on a solid support and, as the mobile phase, a polar organic solvent to provide an eluate free of bacterial contaminants; and

g) subjecting the reversed-phase HPLC eluate to diafiltration to yield a retentate of pure polypeptide R32LA.

9. A pharmaceutical composition comprising an immunogenic polypeptide which comprises four or more tandem repeat units of the P.falciparum CS protein purified according to any one of claims 1 to 8 and a pharmaceutical carrier.

10. A composition according to claim 9 in the form of a vaccine.